(19) Europäisches Patentamt
European Patent Office
Office européen des brevets

(11) **EP 2 426 479 B1**

(12) **EUROPEAN PATENT SPECIFICATION**

(45) Date of publication and mention
of the grant of the patent:
**15.07.2015 Bulletin 2015/29**

(51) Int Cl.:
**G01N 21/35** (2014.01)      **G01N 33/20** (2006.01)

(21) Application number: **10769591.8**

(86) International application number:
**PCT/JP2010/056014**

(22) Date of filing: **01.04.2010**

(87) International publication number:
**WO 2010/125892 (04.11.2010 Gazette 2010/44)**

(54) **METHOD FOR ANALYZING OXYGEN IN STEEL**

VERFAHREN ZUR SAUERSTOFFANALYSE BEI STAHL

PROCÉDÉ POUR ANALYSER DE L'OXYGÈNE DANS DE L'ACIER

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **30.04.2009 JP 2009110824**

(43) Date of publication of application:
**07.03.2012 Bulletin 2012/10**

(73) Proprietor: **Nippon Steel & Sumitomo Metal
Corporation
Tokyo 100-8071 (JP)**

(72) Inventor: **ARAI Masahiro
Osaka-shi
Osaka 541-0041 (JP)**

(74) Representative: **Jackson, Martin Peter
J A Kemp
14 South Square
Gray's Inn
London WC1R 5JJ (GB)**

(56) References cited:
**EP-A1- 1 367 391      EP-A2- 1 160 561
JP-A- 2000 193 657    JP-A- 2002 328 125**

- **SUGIMOTO M ET AL: "Surface variation caused
by vacuum arc cleaning of organic contaminant",
THIN SOLID FILMS, ELSEVIER-SEQUOIA S.A.
LAUSANNE, CH, vol. 506-507, 26 May 2006
(2006-05-26), pages 337-341, XP025005719, ISSN:
0040-6090, DOI: 10.1016/J.TSF.2005.08.078
[retrieved on 2006-05-26]**
- **TAKEDA K ET AL: "Effects of pressure on the
cleaning action of cathode spot in low vacuum",
THIN SOLID FILMS, ELSEVIER-SEQUOIA S.A.
LAUSANNE, CH, vol. 407, no. 1-2, 22 March 2002
(2002-03-22), pages 163-168, XP004352916, ISSN:
0040-6090, DOI: 10.1016/S0040-6090(02)00032-9**

## Description

Technical Field

The present invention relates to a method for rapid analysis of oxygen in steel with high accuracy. The method can be applied to steel making processes which comprises a series of steps of: preparing a sample for composition analysis by collecting an aliquot of molten steel undergoing refining, solidifying it into a block-shaped sampling material and machining the material, analyzing the concentration of elements contained in the sample and adjusting the composition of the molten steel based on the results of analysis.

Background Art

By reflection of the recent trend of increasing the strength and quality of steel products, there have been many attempts at controlling the type, composition, and form of inclusions which develop in steel. For example, in high strength line pipes, in order to avoid hydrogen induced cracking in the environment of use by suppressing the formation of inclusions which become the starting points of cracks, calcium is added in the final stage of refining to fix sulfur as CaS or to form calcium aluminates ($mCaO-nAl_2O_3$). In high purity steels used as a material for bearings or the like, it is necessary to reduce the quantity of inclusions themselves to an extremely low level. In the former case, it is necessary for the added amount of calcium to be optimized in accordance with the oxygen concentration in molten steel. In the latter case, the oxygen concentration itself can be an index of material properties. Accordingly, analyzing the oxygen concentration in molten steel during refining with high accuracy is an important goal of manufacturing technology of steel products in order to improve their performance.

In steel materials in general, alloy design of steel by which various elements are added in suitable amounts is employed, and during actual manufacture of steel having the intended properties based on the design with certainty, the concentration of each element at the time of melting during steelmaking is controlled to be within a prescribed range so as to stabilize the properties of the product. In addition, analysis of the composition of molten steel is carried out during refining in order to ascertain the concentrations of some elements in interest, and the composition is suitably adjusted based on the results of analysis. Therefore, it is essential not only that the results of analysis be accurate, but that analysis be rapid, from the standpoint of avoiding decreases in throughput and energy losses caused by waiting until the results of analysis are obtained.

Accordingly, if the concentration of elements contained in steel can be rabidly and accurately analyzed during refining in a steel manufacturing process for manufacturing a steel having desired properties by addition of various elements, it is possible to manufacture a steel having stable properties at a low cost and with little environmental impact by carrying out suitable adjustment of the composition based on the results of analysis.

The oxygen concentration in steel ranges from several ppm to several hundred ppm. In order to accurately analyze the oxygen concentration in this range, an oxygen elemental analyzer which operates on the principle of heating and fusing in an inert gas combined with infrared absorption detectors is exclusively used. However, in order to obtain a sample for analysis, it is necessary to prepare a sample by machining a steel block to prescribed dimensions and then subject the sample to pretreatment such as chemical polishing, electrolytic polishing, or grinding using a grinder, a file, or the like in order to remove an oxide film from the surface of the sample. The machining and pretreatment have the problems that (1) they involve complicated operations and are time consuming, (2) the extent of removal of an oxide fihn varies depending upon the sample, the treatment, or the operator, which produces variations in the result of analysis and (3) the surface of the sample from which an oxide film is removed soon reoxidizes, thereby causing the analyzed value to increase. Therefore, a sufficient speed and accuracy of analysis could not be achieved and it was difficult to use this method for analyzing oxygen in molten steel during refining.

Patent Document 1 discloses a method for the analysis of a minute amount of oxygen which achieves a total analysis time from pretreatment of a sample until analysis of three minutes by performing preheating the sample at a low temperature to remove an oxide film from the surface of the sample. However, belt polishing which is carried out in pretreatment and a deoxidation reaction which occurs in the preheating do not have high reproducibility, resulting in variations in the results of analysis. Therefore, this method has the problem that the accuracy of analysis is not necessarily improved compared to conventional methods. Furthermore, since it is time consuming to machine the sample to a shape which can be subjected to belt polishing, it is not possible to apply the method to the analysis of oxygen in molten steel during refining.

Patent Document 2 discloses a method and apparatus for analyzing a minute amount of oxygen in metal characterized by dropping a sample directly from a pretreatment chamber into a heating chamber containing a graphite crucible. However, the method employs inert gas sputtering as pretreatment. Therefore after a sample is introduced into the pretreatment chamber, the pretreatment chamber is evacuated in order to then fill with the inert gas to reach a pressure at which sputtering is possible. A certain time is required for initially evacuating the pretreatment chamber to a high vacuum. Although the document does not disclose the time required for analysis including pretreatment, it is clear that the method cannot be used to analyze oxygen in molten steel during refining

A similar method, using a glow discharge or an arc discharge as pretreatment, is disclosed in EP 1160561.

Prior Art Documents

Patent Document 1: JP H06-148170 A (1994)
Patent Document 2: JP H10-073586 A (1998)
Patent Document 3: JP 2002-328125 A
Patent Document 4: JP H10-311782 A (1998)

Summary of the Invention

During the course of investigating the present invention, a careful examination of material properties and requirements of a refining process showed that the accuracy of analysis of oxygen in molten steel during refining and the time required for analysis are as follows.

(1) Accuracy of Analysis For steel having an oxygen concentration of at most 50 ppm, the error should be at most $\pm 2$ ppm and preferably at most $\pm 1$ ppm.
(2) Time Required for Analysis

After receipt of a steel block, the time required for sample processing, pretreatment for the purpose of cleaning, and determination of the oxygen concentration (referred to below as the time for analysis) is at most 5 minutes and preferably at most 4 minutes.

In the prior art, highly accurate and rapid analysis satisfying these requirements with respect to accuracy and time was not possible.

Accordingly, the object of the present invention is to provide a method capable of rapidly analyzing oxygen in molten steel during refining with high accuracy which satisfies the above requirements.

As a result of various investigations, the present inventors discovered a highly accurate and rapid method for analyzing oxygen which can be applied to analysis of oxygen in molten steel during refining and which is the combination of a simple and rapid sample processing method, a rapid and highly reproducible sample pretreatment method and a highly accurate analysis method. Together with the optimization of the conditions for each of these constituent methods, the following invention has been completed.

(1) A method of analyzing oxygen in steel which comprises heating a steel sample loaded in a graphite crucible to fuse in an inert gas and measuring the oxygen concentration in the sample from the degree of infrared absorption of one or both of generated carbon monoxide and carbon dioxide, **characterized in that** the sample is a piece obtained by machining a steel block taken from molten steel so that its height is at least 1.5 mm and at most 7 mm and the ratio (S/V) of its surface area S to its volume V is at least 1.05 mm$^{-1}$ and at most 1.30 mm$^{-1}$, that vacuum arc plasma treatment is carried out as pretreatment for removing an oxide film from the surface of a sample and cleaning the surface under conditions of a vacuum at the start of arc plasma discharge of at least 5 Pa and at most 35 Pa and an arc plasma output current of at least 15 A and at most 55 A with a total number of times that vacuum arc plasma discharge is performed being at most 4 times and a total treatment time being at least 0.2 seconds and at most 1.2 seconds, and that the sample is then directly loaded, without contacting the atmosphere, into the graphite crucible which is waiting after cleaning has been performed thereon by heating at a temperature higher than the analysis temperature followed by lowering the temperature to the analysis temperature, whereby after receipt of the steel block, the time taken for sample processing pretreatment for the purpose of cleaning and determination of the oxygen concentration is at most 5 minutes.

According to the present invention, highly accurate and rapid analysis of oxygen is made possible, Therefore, the analysis can be applied to steel making processes which comprises a series of steps of obtaining a solidified block from molten steel during refining, preparing a sample for composition analysis by machining the block, analyzing the concentrations of elements contained in the sample, and adjusting the composition of the molten steel based on the results of analysis. As a result, refining conditions can be adjusted in accordance with the oxygen concentration of molten steel. In particular, stable manufacture of high quality steel such as steels for line pipes or bearings having properties which depend on the amount, form, and composition of inclusions present in the steel can be realized.

Brief Explanation of the Drawings

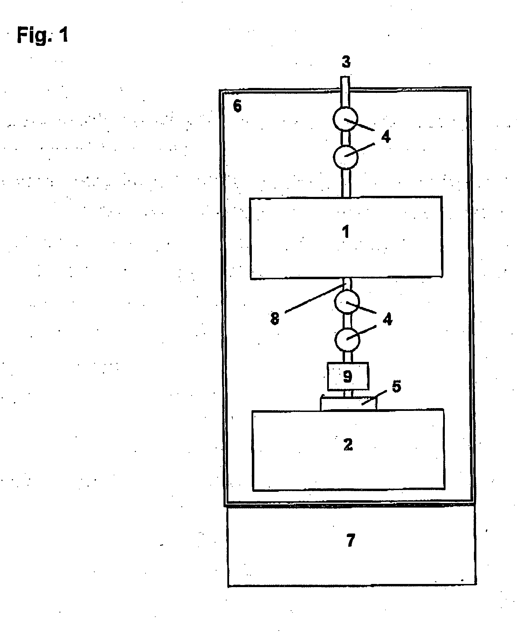

Figure 1 schematically shows an apparatus for analyzing oxygen in steel according to the present invention.

Modes for Carrying Out the Invention

Below, the best mode of a method for analyzing oxygen in steel according to the present invention will be explained while referring to the drawing.

Figure 1 schematically shows an apparatus for analyzing oxygen in steel according to the present invention. Among the technological components which are combined in the present invention, vacuum arc plasma treatment is selected as a sample pretreatment method which is rapid and has high reproducibility. For example, the method and apparatus disclosed in Patent Document 3 for pretreating a sample for analysis of components in metal may be employed, A sample can be inserted from an inlet 3 for a sample to be treated into a sample pretreatment unit 1 which has been maintained at a vacuum through isolating valves 4 without a significant change in the vacuum. An oxide film on the surface of the sample is then removed by vacuum arc plasma treatment in a few seconds. In order to automatically transport the sample, the shape of the sample in this unit is limited to a circular column or a block (a parallelepiped). Because the sample is placed on a sample base during treatment, the surface of the sample which contacts the sample base is not treated. Therefore, it is necessary to invert the sample and to continue the plasma treatment. Namely, it is necessary to perform vacuum arc plasma discharge at least two times on one sample, As the number of times that discharge is performed increases, the sample comes to be heated for a long period of time and a surface of the sample from which an oxide film was once removed is reoxidized. Accordingly, in order to remove an oxide film from the surface of the sample with certainty, accuracy and good reproducibility and to guarantee the accuracy of analysis necessary for refining, it is necessary to perform vacuum arc plasma treatment under the following conditions.

(1) Vacuum: At least 5 Pa and at most 35 Pa
Increasing the vacuum promotes the reaction which removes an oxide film from the surface of a sample by vacuum arc plasma. However, if the vacuum exceeds 35 Pa, there is marked reoxidation which accompanies an increase in the temperature of the sample, which is undesirable. On the other hand, if the vacuum is lower than 5 Pa, the reaction for removing the oxide film itself no longer progresses, which is not desirable. Accordingly, an optimal vacuum exists.
A pressure controller which controls a vacuum exhaust valve and a gas introducing valve is preferably provided so as to maintain the vacuum at a constant value during Treatment.
(2) Arc plasma output current: At least 15 A and at most 55 A
(3) Treatment time: As stated below, the total treatment time for one sample is at least 0.2 seconds and at most 1.2 seconds.
(4) Number of times of treatment: As stated below, the total number of times of the vacuum arc plasma treatment which is performed on one sample is made at most 4 times.

The sample after treatment is finally introduced into a graphite crucible through an inlet 5 for a pretreated sample provided on an analyzer 2 without contacting the atmosphere. The sample pretreatment chamber and the sample inlet of the analyzer are connected by a connecting pipe 8, the interior of which is made a vacuum or replaced by an inert gas. Taking into consideration the difference in specific gravity with respect to air, argon is preferred as an inert gas from the standpoints of replacing the gas inside the connecting pipe with certainty and preventing reoxidation of the sample after treatment and also from the standpoint of economy. With an apparatus having the structure disclosed in Patent Document 3, a pretreated sample is transferred to an oxygen elemental analyzer. However, in view of speed which is one of the objects of the present invention, the sample pretreatment unit 1 and the oxygen elemental analyzer 2 are disposed vertically atop one another and a method of transferring the sample by letting it drop through the connecting pipe 8 is employed. Namely, an apparatus having a structure like that shown in Figure 1 is employed.

With an apparatus having a structure according to the present invention, the oxygen elemental analyzer 2 is disposed close to the floor, and this interferes with maintenance operations of the apparatus such as cleaning of the interior of the analyzer 2 and replacement of an impurity adsorbent for the gas. Therefore, the entire apparatus which is incorporated into a frame 6 is mounted on a lifter 7 in order to allow it to be raised and lowered, and at the time of maintenance operations, the entire apparatus is raised so as to facilitate operation. There are no particular limitations on a system for driving the lifter 7. However, as the overall apparatus is considerably heavy, an automated hydraulic system is preferred from the standpoint of ease of operation. A structure is preferably employed which takes into consideration safety by covering the movable portions of the lifter with a stretchable material so that an operator does not become caught therein.

In contemplation of situations in which the oxygen elemental analyzer 2 is out of operation and cannot be used or in which a pretreated sample which is awaiting analysis is analyzed in a separate analyzer, an outlet 9 for a pretreated sample is preferably provided along the connecting pipe 8 between the sample pretreatment unit 1 and the oxygen elemental analyzer 2.

Among the technological components which are combined by the present invention, a method in which a steel block is cut into a slice having a height (thickness) of at least 1.5 mm and at most 7 mm and the slice is then punched into the shape of a columnar piece is selected as a simple and rapid method of machining a sample for analysis from a steel block taken from molten steel. Specifically, it is possible to use the method and apparatus for preparing a sample for analysis disclosed in Patent Document 4. In order to remove an oxide film from the surface of a sample with certainty, accuracy and good repeatability, it is necessary for the sample to have a shape such that the ratio S/V of the surface area S to the volume V calculated from the diameter of the bottom surface of the sample and its height satisfies the following Equation (1):

$$1.05 \ \text{mm}^{-1} \leq \text{S/V} \leq 1.30 \ \text{mm}^{-1}$$

Although the reason why it is necessary to satisfy this formula has not yet been adequately elucidated, it is assumed that the above equation corresponds to the fact that a position which is suitable for efficient arc plasma treatment is limited by the spatial distribution of arc plasma which depend upon the geometry of an arc plasma treatment chamber, such as the shape of an electrode.

Among the technological components which are combined by the present invention, an oxygen elemental analyzer, which operates on the principle of inert gas fusion and infrared detection is selected as a method of analyzing oxygen in steel with high accuracy. In this analysis method, a graphite crucible is used to function as both a sample holder and a source of a deoxidizer (carbon) for the sample.

Prior to analysis, in order to remove oxygen and contaminants adsorbed by the surface of the crucible, so-called "prebaking" or outgassing of the crucible is carried out in which only the crucible is heated, prior to analysis, to a temperature somewhat higher than that employed during analysis. Prebaking can diminish the effect of oxygen, carbon monoxide, or carbon dioxide which is generated from the graphite crucible and which affects the value of analysis. During analyzing oxygen in steel using a commercially available oxygen elemental analyzer, a crucible and hence a sample is normally heated to a temperature of around 1800 - 2200°C. In order to realize the high accuracy of analysis required by the present invention, the graphite crucible may be prebaked by heating at a temperature of at least 100°C higher than the temperature for analysis for at least 15 seconds.

In the case of a commercially available oxygen elemental analyzer, first a sample is loaded into the analyzer, and then the atmosphere surrounding the sample is purged by helium gas which serves as a carrier gas. During the purge, exchange of the crucible, cleaning of electrodes, and prebaking of the crucible are carried out, Accordingly, a comparatively long time is required from the time when the sample is introduced until the results of analysis are obtained. By performing exchange of the crucible, cleaning of electrodes, and prebaking of the crucible in advance and then introducing a sample which has undergone cleaning pretreatment into an analyzer which is ready to perform analysis, a speedy analysis satisfying the demanded time for analysis can be realized.

Normally, when carrying out oxygen analysis, it is necessary to precisely weigh a sample in order to convert the detected amounts of gases into the oxygen concentration in the sample. The result of evaluation of the change in the weight of a sample between before and after vacuum arc plasma treatment showed that the decrease in weight due to the treatment was at most around 1 mg, although there were some variations depending on the shape of the sample or the degree of surface oxidation. Thus, it was found that with a sample of 0.5 1.0 g in weight the error caused by the decrease in weight is of a level that can be ignored for practical purposes. Therefore, when carrying out the present invention, a sample for analysis which has been previously weighed after machining undergoes vacuum arc plasma treatment and is directly introduced into the oxygen elemental analyzer without contacting the atmosphere.

Examples

[Example 1]

Control samples for analyzing gas in steel of Japan Steel Certified Reference Materials (JSS) identified as GS-3c (oxygen concentration [O] = 34.6 ppm) and GS-5c (oxygen concentration [O] = 125 ppm) were machined to obtain circular column-shaped samples for analysis (S/V = 1.07 mm$^{-1}$ - 1.60 mm$^{-1}$). The latter samples were used only for preparing a calibration curve. After being weighed, each sample underwent pretreatment in a sample pretreatment unit which could perform vacuum arc plasma treatment (Model AP1 of Stec Co., Ltd.), and it was then transferred in an atmosphere purged by argon and introduced into an oxygen elemental analyzer (Model TCH-600 of LECO Corporation) to analyze the oxygen concentration of the sample. The electrical power applied at the time of prebaking of the crucible and at the time of analysis was 5.0 kW (corresponding to approximately 2200°C) and 4.0 kW (corresponding to approximately 2000°C), respectively. The average of two measured values was used as the result of analysis. Vacuum arc plasma treatment was carried out to analyze [O] while using the following standard test conditions except for those specifically specified

Vacuum at the start of discharge: 20 Pa

Treatment time: 0.4 seconds

Output current: 30 At

Total number of times of treatment: 2 times (1 time with respect to each of the front and rear surfaces of the sample)

In vacuum arc plasma treatment, prior to carrying out discharge toward a sample, discharge was carried out with the sample being removed in order to perform "cleaning discharge". The conditions for cleaning discharge were the same as the above-described discharge conditions.

(1) Vacuum at the start of vacuum arc plasma discharge

Oxygen analysis was carried out on GS-3c samples which had undergone the above-described treatment while varying the vacuum at the start of vacuum arc plasma discharge. As a result, at a vacuum of at least 5 Pa and at most 35 Pa, the error was $\pm 2$ ppm and the desired accuracy of analysis was obtained.

Table 1

| Vacuum (Pa) | Analyzed value of [O] (ppm) | Error (ppm) | Remarks |
|---|---|---|---|
| 10 | 35.7 | 1.1 | This invention |
| 20 | 34.6 | 0 | This invention |
| 30 | 33.8 | -0.8 | This invention |

(2) Duration of vacuum arc plasma discharge

Oxygen analysis was carried out on GS-3c samples which had undergone the above-descibed treatment while varying the duration of vacuum arc plasma discharge. Discharge was carried out for the same length of time on the front and rear surfaces of the samples. When the total discharge time was at least 0.2 seconds and at most 1.2 seconds, the error was within $\pm 2$ ppm, and the desired accuracy of analysis was obtained. The sample temperature after treatment was particularly high when the treatment time was 1.4 seconds or longer, indicating that reoxidation of the sample surface occurred.

Table 2

| Total discharge time (s) | Analyzed value of [O] (ppm) | Error (ppm) | Temperature of sample after treatment (°C) | Remarks |
|---|---|---|---|---|
| 0.2 | 33.4 | -0.8 | 27 | This invention |
| 0.4 | 35.1 | 0.5 | 31 | This invention |
| 0.6 | 34.4 | -0.2 | 34 | This invention |
| 0.8 | 34.6 | 0 | 34 | This invention |
| 1.0 | 34.4 | -0.2 | 43 | This invention |
| 1.2 | 35.6 | 1.0 | 44 | This invention |
| 1.4 | 39.4 | 4.8 | 59 | Comparative |
| 1.6 | 46.9 | 12.3 | 68 | Comparative |

(3) Number of times of discharge

Oxygen analysis was carried out on GS-3c samples which had undergone the above-described treatment while varying the number of times that vacuum arc plasma discharge was performed. Discharge was carried out the same number of times on the front and rear surfaces of the samples. When the total number was at most 4 times, the error was at most ±2 ppm and the desired accuracy of analysis was obtained.

Table 3

| Total number of discharges | Analyzed value of [O] (ppm) | Error (ppm) | Remarks |
|---|---|---|---|
| 2 | 32.8 | -1.8 | This invention |
| 4 | 36.5 | 1.9 | This invention |
| 6 | 39.5 | 4.9 | Comparative |
| 8 | 37.2 | 2.6 | Comparative |

(4) Arc plasma output current

Oxygen analysis was carried out on GS-3c samples which had undergone the above-described treatment while varying the output current during vacuum arc plasma treatment. In a range of at least 15 A up to at most 55 A, the error was at most ±2 ppm and the desired accuracy of analysis was obtained.

Table 4

| Output current (A) | Analyzed value of [O] (ppm) | Error (ppm) | Remarks |
|---|---|---|---|
| 10 | 39.1 | 4.5 | Comparative |
| 20 | 34.5 | -0.1 | This invention |
| 30 | 36.1 | 1.5 | This invention |
| 40 | 34.4 | -0.2 | This invention |
| 50 | 36.0 | 1.4 | This invention |
| 60 | 37.6 | 3.0 | Comparative |

(5) Atmosphere for holding the sample after vacuum arc plasma treatment

Oxygen analysis was carried out on GS-3c samples while varying the atmosphere in which the samples were held in the period from the completion of vacuum arc plasma treatment until they were introduced into the oxygen elemental analyzer. For samples exposed to the atmosphere (air) after the treatment the error exceeded ±2 ppm and the desired accuracy of analysis was not obtained.

Table 5

| Atmosphere after treatment | Analyzed value of [O] (ppm) | Error (ppm) | Remarks |
|---|---|---|---|
| argon | 34.6 | 0 | This invention |
| air | 37.0 | 2.4 | Comparative |

(6) Shape of sample

Samples which differed with respect to shape and oxygen content were prepared, and after vacuum arc plasma treatment was carried out under standard conditions, the oxygen concentrations in the samples were analyzed. Separately, a sample which was taken from a location of the same steel block which is as close to the previous sample as possible underwent analysis by a conventional method. Namely, the surface of the sample was polished with a file and subjected to oxygen analysis. The resulting analytical value was used as the oxygen concentration contained in the sample.

When the ratio S/V of the surface area S and the volume V of the sample was within the range of the present invention, namely, when it was at least 1.05 mm$^{-1}$ and at most 1.30mm$^{-1}$, the analyzed value well corresponded to the oxygen concentration and the error was at most +-2 ppm, so the desired accuracy of analysis was obtained.

Table 6

| Sample dimensions (mm) | Surface area S (mm$^2$) | Volume V(mm$^3$) | S/V (mm$^{-1}$) | [O] concentration (ppm) | Analyzed value of [O] (ppm] | Error (ppm) | Remarks |
|---|---|---|---|---|---|---|---|
| 6 x 6 x 5 | 192 | 180 | 1.07 | 38.3 | 38.0 | -0.8 | This invention |
| 6 x 6 x 5 | 192 | 180 | 1.07 | 41.0 | 40.8 | -0.2 | This invention |
| 7dia. x 3.8 t | 161 | 146 | 1.10 | 22.0 | 21.4 | 0.6 | This invention |
| 6 dia. x 4 t | 132 | 113 | 1.17 | 34.6 | 35.2 | 0.6 | This invention |
| 5 dia. x 4.5 t | 110 | 88.4 | 1.25 | 34.6 | 33.6 | -1.0 | This invention |
| 6 dia. x 2.5 t | 104 | 70.7 | 1.47 | 38.3 | 53.1 | 14.8 | Comparative |
| 6 dia. x 2.5 t | 104 | 70.7 | 1.47 | 41.0 | 61.9 | 20.9 | Comparative |

From the above results, it is clear that of the objects of the present invention, the object of obtaining accurate analysis is satisfied by treating and analyzing a sample under the conditions prescribed by the claims.

Example 2

A steel block taken from molten steel was prepared and subjected to oxygen analysis under the standard conditions set forth in Example 1. The results are shown in Table 7.

Condition 1 was a conventional method. Namely, the surface of a sample which was machined from a steel block was polished with a file and subjected to oxygen analysis. A long time was required for processing the sample and pretreatment, so this condition did not satisfy the requirements concerning the time required for analysis.

In Conditions 2 and 3, a sample was obtained by automated machining (preparation of a slice by cutting followed by punching of the slice), and vacuum arc plasma treatment was applied as pretreatment. Only Condition 3, namely, the case in which exchange of a crucible, cleaning of electrodes, and prebaking of the crucible (indicated in the table as "prior operation of crucible, etc,") were carried out in advance had a time for analysis of at most 4 minutes and satisfied the requirements.

Variations in the time required for analysis depending upon the analyzer (the manufacturer and the model) and upon the details of the analysis conditions do not impair the effect of the present invention that the time for analysis is shortened by previously performing exchange of a crucible and prebaking of the crucible.

Table 7

| Condition | Prior operation of crucible, etc. | Time required | | | | | [O] (ppm) | | Remarks |
|---|---|---|---|---|---|---|---|---|---|
| | | Sample processing | Weighing | Pretreatment | Analysis | Total | Analytical value | Error | |
| 1 | no | 10 min | 15 sec | 1 min 30 sec | 4 min 30 sec | 15 sec | 22.0 | - | Comparative (conventional method) |
| 2 | no | 1 min 30 sec | 15 sec | 30 sec | 4 min 30 sec | 6 min 45 sec | 23.3 | 1.3 | Comparative |
| 3 | yes | 1 min 30 sec | 15 sec | 30 sec | 1 min 40 sec | 3 min 55 sec | 20.4 | -1.6 | This invention |

As described above, by applying the present invention to a process during steelmaking which comprises a series of steps of obtaining a solidified block from molten steel during refining, preparing a sample for composition analysis by machining the steel block, analyzing the concentration of oxygen contained in the sample, and adjusting the composition of the molten steel based on the results of analysis, the present invention can achieve the goals of improving the accuracy of analysis and shortening the analysis time both demanded of oxygen analysis technology.

## Claims

1.  A method of analyzing oxygen in steel which comprises heating a steel sample loaded in a graphite crucible to fuse in an inert gas and measuring the oxygen concentration in the sample from the degree of infrared absorption of one or both of generated carbon monoxide and carbon dioxide, **characterized in**
    **that** the sample is a piece obtained by machining a steel block taken from molten steel so that its height is at least 1.5 mm and at most 7 mm and the ratio (S/V) of its surface area S to its volume V is at least 1.05 mm$^{-1}$ and at most 1.30 mm$^{-1}$,
    **that** vacuum arc plasma treatment (1) is carried out as pretreatment for removing an oxide film from the surface of a sample and cleaning the surface under conditions of a vacuum at the start of arc plasma discharge of at least 5 Pa and at most 35 Pa and an arc plasma output current of at least 15 A and at most 55 A with a total number of times that vacuum arc plasma discharge is performed being at most 4 times and a total treatment time being at least 0.2 seconds and at most 1.2 seconds, and
    **that** the sample is then directly loaded, without contacting the atmosphere, into the graphite crucible (5) which is waiting after cleaning has been performed thereon by heating at a temperature higher than the analysis temperature followed by lowering the temperature to the analysis temperature,
    whereby after receipt of the steel block, the time taken for sample processing pre-treatment for the purpose of cleaning and determination of the oxygen concentration is at most 5 minutes.

## Patentansprüche

1.  Verfahren zur Analyse von Sauerstoff in Stahl, das Folgendes umfasst: Erhitzen einer Stahlprobe, die in einen Graphitschmelztiegel geladen ist, um ein Inertgas einzuschmelzen und Messen der Sauerstoffkonzentration in der Probe ausgehend von dem Grad der Infrarotabsorption des erzeugten Kohlenmonoxids und/oder des erzeugten Kohlendioxids, **gekennzeichnet durch** Folgendes:

    die Probe ist ein Stück, das **durch** Bearbeitung eines Stahlblocks enthalten wurde, der einer Stahlschmelze entnommen wurde, so dass seine Höhe mindestens 1,5 mm und höchstens 7 mm und das Verhältnis (S/V) seines Oberflächenbereichs S zu seinem Volumen V mindestens 1,05 mm$^{-1}$ und höchstens 1,30 mm$^{-1}$ beträgt, eine Vakuumlichtbogenplasmabehandlung (1) wird als Vorbehandlung zum Entfernen eines Oxidfilms von der Oberfläche einer Probe durchgeführt und eine Reinigung der Oberfläche wird durchgeführt unter Bedingungen eines Vakuums zu Beginn der Lichtbogenplasmaentladung von mindestens 5 Pa und höchstens 35 Pa und mit einem Lichtbogenplasmaausgangsstrom von mindestens 15 A und höchstens 55 A mit einer Gesamtzahl von Malen, mit der die Vakuumlichtbogenplasmaentladung durchgeführt wird, von höchstens 4 und einer Gesamtbehandlungszeit von mindestens 0,2 Sekunden und höchstens 1,2 Sekunden und
    die Probe wird daraufhin direkt und ohne Kontakt mit der Atmosphäre in den Graphitschmelztiegel (5) geladen, der wartet, nachdem die Reinigung daran durchgeführt wurde, indem auf eine Temperatur erhitzt wurde, die höher ist als die Analysetemperatur, gefolgt von einem Absenken der Temperatur auf die Analysetemperatur, wobei nach Erhalt des Stahlblocks die Zeit für die Probenverarbeitungsvorbehandlung zum Zweck der Reinigung und der Bestimmung der Sauerstoffkonzentration höchstens 5 Minuten beträgt.

## Revendications

1.  Procédé d'analyse de l'oxygène dans l'acier qui comprend le chauffage d'un échantillon d'acier chargé dans un creuset en graphite pour une fusion dans un gaz inerte et la mesure de la concentration en oxygène dans l'échantillon à partir du degré d'absorption d'infrarouge de l'un ou des deux éléments parmi le monoxyde de carbone et le dioxyde de carbone généré, **caractérisé en ce que**
    l'échantillon est une pièce obtenue par usinage d'un bloc d'acier pris dans de l'acier fondu de sorte que sa hauteur soit d'au moins 1,5 mm et d'au plus 7 mm et que le rapport (S/V) entre sa surface spécifique S et son volume V soit d'au moins 1,05 mm$^{-1}$ et d'au plus 1,30 mm$^{-1}$,

**en ce qu'**un traitement par arc à plasma sous vide (1) conduit en guise de prétraitement pour éliminer un film d'oxyde de la surface d'un échantillon et nettoyer la surface dans les conditions d'un vide au début d'une décharge d'arc à plasma d'au moins 5 Pa et d'au plus 35 Pa et d'un courant de sortie d'arc à plasma d'au moins 15 A et d'au plus 55 A, avec un nombre d'exécutions de décharge d'arc à plasma sous vide d'au plus 4 et un temps total de traitement d'au moins 0,2 seconde et d'au plus 1,2 seconde, et

**en ce que** l'échantillon est ensuite directement chargé, sans contact avec l'atmosphère, dans le creuset en graphite (5) qui est en attente après la conduite d'un nettoyage de celui-ci par chauffage à une température supérieure à la température d'analyse, puis un abaissement de la température à la température d'analyse,

moyennant quoi, après réception du bloc d'acier, le temps nécessaire au prétraitement d'échantillon en vue du nettoyage et de la détermination de la concentration en oxygène est d'au plus 5 minutes.

**Fig. 1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1160561 A
- JP H06148170 A
- JP H10073586 A
- JP 2002328125 A
- JP H10311782 A